# FASCICULE DE BREVET EUROPEEN

(11) **EP 1 601 768 B1**
(45) Date de publication et mention de la délivrance du brevet: **09.05.2007**
(21) Numéro de dépôt: 04717665.6
(22) Date de dépôt: 05.03.2004
(51) Int. Cl.: C12N 15/12, A61K 38/17, C07K 14/435, A01H 4/00, A61P 31/00

(54) **PEPTIDE ANTIMICROBIEN APPELE HALOCYNTIN, GENE CODANT LEDIT PEPTIDE, VECTEUR, ORGANISME TRANSFORME ET COMPOSITION LE CONTENANT.**
ANTIMIKROBIELLES PEPTID, GENANNT HALOCYTIN; DAFÜR KODIERENDES GEN, VEKTOR, TRANSFORMIERTER ORGANISMUS UND ZUSAMMENSETZUNG DIE ES ENTHALTEN
ANTI-MICROBIAL PEPTIDE KNOWN AS HALOCYNTIN, GENE CODING FOR SAID PEPTIDE, VECTOR, TRANSFORMED ORGANISM AND COMPOSITION CONTAINING SAID PEPTIDE

(30) Priorité: 05.03.2003 FR 0302715
(43) Date de publication de la demande: 07.12.2005
(73) Titulaire: CENTRE NATIONAL DE LA RECHERCHE SCIENTIFIQUE (CNRS), 75794 Paris Cedex 16 (FR); Université de Perpignan, 66860 Perpignan (FR)
(72) Inventeur: MITTA, Guillaume, F-66540 Baho (FR); GALINIER, Richard, F-66100 Perpignan (FR); BANAIGS, Bernard, F-66140 Canet-en-Roussillon (FR); LASSERRE, Eric, F-66180 Villeneuve-De-la-Raho (FR)
(74) Mandataire: Breese Derambure Majerowicz
(86) Numéro de dépôt international: PCT/FR2004/000536
(87) Numéro de publication internationale: WO 2004/081024

(56) Documents cités:
- WO-A-98/20028
- WO-A-98/38309
- TOSSI A ET AL: "Molecular diversity in gene-encoded, cationic antimicrobial polypeptides." CURRENT PHARMACEUTICAL DESIGN. NETHERLANDS 2002, vol. 8, no. 9, 2002, pages 743-761, XP008024137 ISSN: 1381-6128
- LEE I H ET AL: "Dicynthaurin: an antimicrobial peptide from hemocytes of the solitary tunicate, Halocynthia aurantium" BBA - GENERAL SUBJECTS, ELSEVIER SCIENCE PUBLISHERS, NL, vol. 1527, no. 3, 15 août 2001 (2001-08-15), pages 141-148, XP004296801 ISSN: 0304-4165
- JANG W S ET AL: "Halocidin: a new antimicrobial peptide from hemocytes of the solitary tunicate, Halocynthia aurantium" FEBS LETTERS, ELSEVIER SCIENCE PUBLISHERS, AMSTERDAM, NL, vol. 521, no. 1-3, 19 juin 2002 (2002-06-19), pages 81-86, XP004362143 ISSN: 0014-5793
- DATABASE UniProt [Online] Thermoanaerobacter thermosulfurogenes 1 février 1997 (1997-02-01), "OrfA (Fragment)" extrait de EBI Database accession no. P74938

## Description

La présente invention concerne un nouveau peptide antimicrobien, ci-après appelé halocyntin, identifié et purifié à partir d'un extrait d'un invertébré marin.

De nombreuses maladies (tuberculose, pneumonies, pathologies urinaires,...), pourtant bien contrôlées depuis l'avènement des antibiotiques, constituent aujourd'hui des pathologies réémergentes, de pronostic souvent fatal, par suite de l'impuissance des antibiotiques classiques.

En effet, autrefois fléau implacable, la tuberculose (causée par le bacille *Mycobacterium tuberculosis*) avait régressé ces quarante dernières années dans les pays industrialisés, grâce à l'amélioration des conditions sociales et à l'application d'un traitement antibiotique efficace. Elle est réapparue sous une forme plus virulente vers le milieu des années 1980 dans de nombreux pays, dont la France et les Etats-Unis. Les "nouvelles" formes du bacille sont résistantes aux antituberculeux classiques (streptomycine, isoniazide, rifampicine) ainsi qu'à d'autres antibiotiques naguère efficaces.

De même, les infections dites nosocomiales fréquemment contractées en milieu hospitalier sont le plus souvent très difficiles à maîtriser en raison des résistances aux antibiotiques disponibles. 20 à 25 % des Pneumocoques isolés en milieu hospitalier en France se sont révélés être résistants aux antibiotiques de la famille des macrolides et 20 à 40 % des *Staphylococcus aureus* isolés en hôpital aux USA sont résistants à la méthicilline.

L'existence et l'apparition constante de bactéries de plus en plus résistantes aux antibiotiques induit une importante demande de la part de l'industrie pharmaceutique et rend absolument nécessaire la découverte de nouvelles familles d'antibiotiques.

Le biotope marin est considéré comme le plus riche des différents habitats du globe mais également comme le moins bien connu des scientifiques. C'est aussi le berceau prébiotique de notre planète (3 milliards d'années d'évolution). Ceci a pour conséquence une diversité d'espèces, de systèmes d'organisation, de formes et de solutions adaptatives. Cette biodiversité est la source d'une formidable chimiodiversité, une source potentielle de composés naturels nouveaux. Les recherches ont déjà conduit à la découverte de substances remarquables, tant par leurs structures que par leurs activités biologiques. Plusieurs milliers de substances sont aujourd'hui répertoriées ; plus de 150 publications, décrivant de nouveaux métabolites secondaires, paraissent chaque année depuis plus de dix ans. Certains de ces métabolites font l'objet d'essais cliniques ou sont déjà commercialisés. D'autres composés, comme les toxines de micro-organismes marins (ciguatoxine, brevetoxine, saxitoxine ou tétrodotoxine) sont des outils de choix en neurophysiologie et en particulier dans l'étude des canaux ioniques.

Aujourd'hui, plus de la moitié des molécules d'origine marine susceptibles d'être utilisées dans le domaine de la santé est destinée au traitement des cancers. En vingt-cinq ans, de 1970 à 1995, plus de 130 substances marines ont été brevetées dans le monde pour leurs propriétés thérapeutiques.

Curieusement le domaine des antibiotiques a été quelque peu oublié. La recherche de nouveaux peptides antibiotiques dans des invertébrés marins n'a été que très récemment abordée. Les peptides antimicrobiens sont des molécules dont la cible est la membrane bactérienne. Par conséquent, pour acquérir une résistance vis-à-vis de ce type de molécules, les micro-organismes devraient changer la composition et l'organisation de leurs lipides membranaires. Cette solution coûteuse d'un point de vue évolutif explique pourquoi la résistance des micro-organismes vis-à-vis de ce type d'antibiotiques n'est que rarement référencée.

Ainsi, les travaux de la Demanderesse ont permis de mettre en évidence et de purifier un nouveau peptide antibiotique, ci-après appelé halocyntin, compte tenu du fait qu'il ne présente aucune homologie de structure primaire par rapport à d'autres molécules décrites dans la littérature et qu'il a été purifié à partir d'une espèce de tunicier, l'ascidie solitaire *Halocynthia papillosa* chez laquelle ce type de molécule n'avait jamais été recherché. L'activité antibactérienne de l'halocyntin a été évaluée au laboratoire : il s'agit d'une molécule bactériolytique essentiellement active contre les bactéries à Gram positif. Cette molécule est particulièrement intéressante de par le fait de son mode d'action particulier. En effet, ce dernier ne permet pas d'induire de résistance de la part des cibles bactériennes.

Dans les séquences peptidiques rapportées ci-après, les acides aminés sont représentés par leur code à une lettre, mais ils peuvent être aussi représentés par leur code à trois lettres selon la nomenclature ci-dessous.

| | | |
|---|---|---|
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | sérine |
| T | Thr | thréonine |
| V | Val | valine |
| W | Trp | tryptophane |
| Y | Tyr | tyrosine |

La présente invention concerne donc un peptide isolé de 26 acides aminés dont la séquence en acides aminés est la suivante: FWGHIWNAVKRVGANALHGAVTGALS (SEQ ID No. 1 dans la liste de séquences en annexe), ses dérivés et ses fragments.

A titre de « dérivés» du peptide de l'invention, on peut citer les peptides qui présentent une modification post-traductionnelle et/ou une modification chimique en particulier une glycosylation, une amidation, une acylation, une acétylation, une méthylation ainsi que les peptides qui portent un groupement protecteur. On entend par " groupement protecteur " selon la présente invention, tout groupement permettant d'éviter la dégradation du peptide de l'invention.

Les dérivés du peptide de l'invention peuvent également être ceux dont un ou plusieurs acides aminés sont des énantiomères, des diastéréoisomères, des acides aminés naturels de conformation D, des acides aminés rares notamment l'hydroxyproline, l'hydroxylysine, l'allohydroxylysine, la 6-N méthylysine, la N-éthylglycine, la N-méthylglycine, la N-éthylasparagine, l'allo-isoleucine, la N-méthylisoleucine, la N-méthylvaline, la pyroglutamine, l'acide aminobutyrique et les acides aminés synthétiques notamment l'ornithine, la norleucine, la norvaline, la cyclohexyl-alanine et les oméga-acides aminés. L'invention couvre également les rétropeptides et les rétroinversopeptides, de même que les peptides dont la chaîne latérale d'un ou plusieurs des acides aminés est substituée par des groupements qui ne modifient pas l'activité antimicrobienne du peptide de l'invention.

A titre de « dérivés » du peptide de l'invention, on entend également des peptides présentant 70%, 75%, 80%, 85%, 90% et/ou 95% d'identité avec le peptide de séquence SEQ ID No. 1 dans la liste de séquences en annexe.

A titre de « fragments » du peptide de l'invention, on entend des fragments d'au moins 7 acides aminés qui présentent une activité antibactérienne. L'activité antimicrobienne des dérivés et des fragments du peptide de l'invention peut être mise en évidence grâce aux tests *in vitro* décrits ci-après dans les exemples.

L'invention concerne aussi un polypeptide isolé comprenant le peptide de l'invention. L'invention envisage plus particulièrement un polypeptide comprenant le peptide de l'invention dont l'une et/ou l'autre des extrémités dudit peptide comprend un ou plusieurs acides aminés nécessaires à son expression et/ou son ciblage dans un organisme hôte. Le polypeptide de l'invention peut présenter un peptide signal ou de transit de façon à diriger la sécrétion du peptide ou polypeptide de l'invention dans un organisme hôte.

Le peptide, ses dérivés et ses fragments tout comme les polypeptides de l'invention peuvent être synthétisés chimiquement selon des techniques connues de l'homme du métier.

L'invention concerne également un polynucléotide isolé caractérisé en ce qu'il code le peptide ou un polypeptide de l'invention. On entend par " polynucléotide " selon la présente invention, une séquence nucléique de type ADN ou ARN, de préférence ADN, notamment double brin. L'homme du métier connaissant le code génétique et la séquence en acides aminés du peptide de l'invention est à même d'isoler un polynucléotide codant le peptide de l'invention par criblage de banques d'acide nucléique en utilisant un ou plusieurs oligonucléotides déduits à partir de la séquence en acides aminés du peptide de l'invention. L'homme du métier a également à sa disposition des logiciels informatiques capables, à partir d'une séquence en acides aminés, de fournir la séquence nucléotidique correspondante (Protéine en code reverse).

L'invention concerne également les polynucléotides isolés qui comprennent des modifications au niveau d'un ou plusieurs nucléotides résultant de la dégénérescence du code génétique et qui codent pour une même séquence d'acides aminés du peptide de l'invention.

L'invention couvre également les polynucléotides isolés codant pour le peptide ou un polypeptide de l'invention et capables de s'hybrider dans des conditions stringentes audit peptide ou auxdits polypeptides. On entend par « conditions stringentes » selon la présente invention, les conditions enseignées par Sambrook et al. (Molecular cloning, 1989, Noland C. ed., New York: Cold Spring Harbor Laboratory Press).

L'invention couvre également les séquences nucléotidiques complémentaires des polynucléotides isolés définis ci-dessus ainsi que les ARN correspondants.

La présente invention concerne également un vecteur de clonage et/ou d'expression caractérisé en ce qu'il contient un polynucléotide selon l'invention pour transformer un organisme hôte et exprimer dans ce dernier le peptide ou un polypeptide de l'invention. De façon avantageuse, le vecteur de clonage et/ou d'expression peut contenir, outre le polynucléotide codant un peptide ou un polypeptide de l'invention, au moins un élément choisi dans le groupe des promoteurs constitutifs, des promoteurs inductibles, des éléments terminateurs.

De façon préférée, ledit vecteur comprend, liés entre eux de façon opérationnelle, un promoteur, un polynucléotide codant le peptide ou un polypeptide de l'invention et un élément terminateur. On entend par " liés entre eux de façon opérationnelle " selon l'invention, des éléments liés entre eux de façon à ce que le fonctionnement d'un des éléments soit affecté par celui d'un autre. A titre d'exemple, un promoteur est lié de façon opérationnelle à une séquence codante lorsqu'il est capable d'affecter l'expression de cette dernière. Les éléments régulateurs de la transcription, de la traduction et de la maturation des peptides que le vecteur peut comprendre sont connus de l'homme du métier et ce dernier est capable de les choisir en fonction de l'organisme hôte dans lequel l'expression ou le clonage doivent être réalisés.

Le vecteur de l'invention est avantageusement choisi parmi un plasmide, un cosmide, un bactériophage et un virus en particulier un baculovirus. De façon préférée, le peptide de l'invention est un vecteur à réplication autonome comportant des éléments permettant son maintien et sa réplication dans l'organisme hôte comme une origine de réplication.

En outre, le vecteur peut comporter des éléments permettant sa sélection dans l'organisme hôte comme, par exemple, un gène de résistance à un antibiotique ou un gène de sélection qui assurent la complémentation avec le gène respectif délété au niveau du génome de l'organisme hôte. De tels vecteurs de clonage et/ou d'expression sont bien connus de l'homme du métier et largement décrits dans la littérature.

L'invention concerne également un organisme hôte caractérisé en ce qu'il est transformé à l'aide d'un vecteur de l'invention. Par « organisme hôte » selon la présente invention, on entend tout organisme mono ou pluricellulaire, inférieur ou supérieur, dans lequel un polynucléotide de l'invention est introduit pour la production d'un peptide ou d'un polypeptide de l'invention. L'homme du métier connaît différentes méthodes pour introduire de façon efficace un polynucléotide dans un organisme hôte et ce, afin que, dans l'organisme hôte, le peptide ou polypeptide codé par ledit polynucléotide soit produit. A titre d'exemple et de façon non exhaustive, cette méthode peut être une électroporation, une lipofection, une transformation biologique d'un végétal en utilisant *A grobacterium tumefasciens,* etc...

Selon une forme préférée de l'invention, l'organisme hôte est un microorganisme tel qu'une levure, une bactérie ou un champignon. La transformation de tels microorganismes permet de produire le peptide de l'invention à échelle semi-industrielle ou industrielle. L'homme du métier connaît de tels microorganismes et sait comment les transformer sans faire preuve d'un effort inventif.

Selon une autre forme de l'invention, l'organisme hôte est une cellule animale telle qu'une cellule de mammifère.

Selon une autre forme de l'invention, l'organisme hôte est une cellule végétale ou une plante. Par « cellule de plante » on entend, selon la présente invention, toute cellule issue d'une plante et pouvant constituer des tissus indifférenciés tels que des cals, des tissus différenciés tels que des embryons, des parties de plantes, des plantes ou des semences. Par « plante », on entend selon l'invention, tout organisme multicellulaire différencié capable de photosynthèse, en particulier des monocotylédones ou des dicotylédones, plus particulièrement des plantes de culture destinées ou non à l'alimentation animale ou humaine.

Par conséquent, l'organisme hôte selon l'invention est choisi parmi les microorganismes, les cellules animales, les cellules végétales et les plantes.

Le peptide de l'invention est également utile pour conférer aux plantes un caractère de résistance aux maladies microbiennes. L'invention concerne donc également une cellule végétale résistante aux maladies microbiennes comprenant un polynucléotide de l'invention et exprimant le peptide ou un polypeptide de l'invention. L'invention se rapporte également à une plante comprenant au moins une cellule végétale résistante aux maladies microbiennes telle que définie ci-dessus.

Enfin, l'invention vise la mise à profit des propriétés antimicrobiennes du peptide de l'invention pour prévenir et/ou traiter les infections microbiennes tant chez l'homme et l'animal que chez les plantes. Dans le cadre de la présente invention, on entend par « propriétés antimicrobiennes », aussi bien des propriétés antibactériennes que des propriétés antifongiques. L'invention concerne donc avantageusement l'utilisation du peptide de l'invention à titre de médicament en thérapie humaine et animale. Elle concerne également l'utilisation du peptide de l'invention pour le traitement des plantes contre les infections microbiennes, en appliquant ledit peptide directement sur les plantes. La présente invention concerne donc l'utilisation d'un peptide ou d'un polypeptide de l'invention comme agent antimicrobien et, plus particulièrement comme agent antibactérien contre les bactéries à Gram positif. La présente invention concerne également l'utilisation d'un peptide ou d'un polypeptide tels que définis précédemment pour la préparation d'une composition antimicrobienne et, plus particulièrement, antibactérienne destinée à lutter contre les bactéries à Gram positif.

L'invention concerne ainsi une composition antimicrobienne comprenant à titre d'agent actif le peptide de l'invention ou un polypeptide de l'invention avantageusement associé dans ladite composition à un véhicule acceptable. La composition agit plus particulièrement contre les bactéries à Gram positif. La composition antimicrobienne de l'invention peut en outre comprendre un autre principe actif tel qu'un autre agent antimicrobien.

Par " véhicule ", on entend selon la présente invention, toute substance qui est ajoutée au peptide ou au polypeptide de l'invention pour favoriser leur transport, éviter leur dégradation substantielle dans ladite composition et préserver leurs propriétés antimicrobiennes. Le véhicule est choisi en fonction du type d'application de la composition. Notamment, lorsque la composition est appliquée à un usage pharmaceutique en santé humaine et animale, l'homme du métier choisira le véhicule pharmaceutiquement acceptable adapté à la voie d'administration de la composition pharmaceutique de l'invention.

Ainsi, les compositions pharmaceutiques selon l'invention sont constituées par au moins le peptide ou le polypeptide de l'invention sous forme libre ou sous forme d'un sel d'addition avec un acide pharmaceutiquement acceptable, à l'état pur ou sous forme d'une composition dans laquelle il est associé à tout autre produit pharmaceutiquement compatible. Les compositions pharmaceutiques selon l'invention peuvent être employées par voie orale, parentérale, rectale ou topique.

A titre de compositions solides pour administration orale, on peut utiliser des comprimés, des pilules, des poudres, etc... où le peptide ou le polypeptide de l'invention sont mélangés à un ou plusieurs diluants inertes classiquement utilisés, et éventuellement à d'autres substances, tels que par exemple un lubrifiant, un colorant un enrobage etc....

A titre de compositions liquides pour administration orale ou oculaire, on peut utiliser des suspensions, des solutions, des émulsions, des sirops pharmaceutiquement acceptables contenant des diluants inertes classiquement utilisés, et éventuellement d'autres substances comme des produits mouillants, édulcorants, épaississant, etc....

Les compositions stériles pour administration parentérale peuvent être des solutions aqueuses ou non, des suspensions ou des émulsions. Comme solvant ou véhicule, on peut employer l'eau, le propylèneglycol, des huiles végétales ou d'autres solvants organiques convenables. Ces compositions peuvent également contenir des adjuvants, comme des agents mouillants, isotonisants, émulsifiants, etc.

Les compositions pour l'administration topique peuvent être par exemple des crèmes, lotions, collutoires, gouttes nasales ou oculaires ou aérosol.

Lorsque la composition antimicrobienne de l'invention est réservée à un usage agrochimique, le véhicule est un véhicule agrochimiquement acceptable adapté à une administration sur les plantes ou à proximité des plantes sans les dégrader.

Dans les compositions antimicrobiennes objet de la présente invention, la quantité de peptide ou de polypeptide objet de l'invention avantageusement utilisée est comprise entre 0,1 et 50 µM. Cependant, il est évident que l'homme du métier saura adapter cette quantité en fonction du type de compositions antimicrobiennes i.e. compositions pharmaceutiques ou compositions agrochimiques et en fonction du mode d'administration desdites compositions.

La présente invention concerne également une méthode pour prévenir et/ou traiter une infection microbienne. La présente méthode comprend l'administration à un sujet d'une quantité efficace d'un peptide, d'un polypeptide, d'un polynucléotide ou d'une composition selon la présente invention. Par « sujet », on entend dans la présente invention tout animal ou tout humain pour lequel une infection microbienne et, plus particulièrement, une infection bactérienne causée par des bactéries à Gram positif a été diagnostiquée mais également tout animal ou tout humain susceptible de souffrir de cette infection.

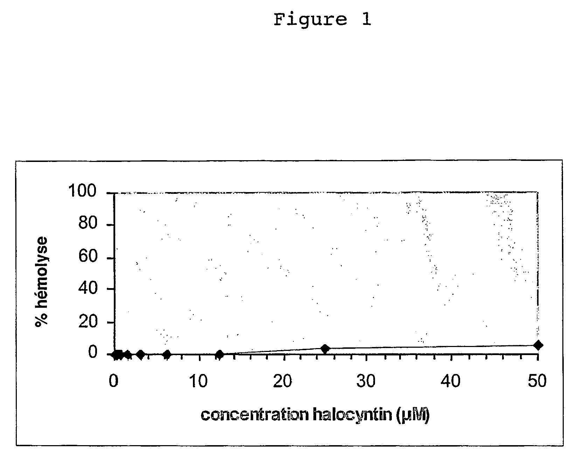

Les exemples qui suivent permettent d'illustrer la présente invention et ne sauraient être interprétés comme limitant sa portée. Ces exemples font plus particulièrement référence à la mise en évidence de l'halocyntin, sa purification et son activité antibactérienne. Les exemples ci-après font référence à la figure 1 en annexe qui représente l'activité hémolytique testée sur les hématies de mouton pour différentes concentrations d'halocyntin.

### I. Isolement de l'halocyntin.

La caractérisation biochimique de cette molécule est complète. L'halocyntin a été isolée à partir des cellules circulantes (hémocytes) de l'ascidie. Elle a été purifiée par chromatographie liquide haute performance (HPLC) en suivant son activité par des tests réalisés *in vitro* tout au long du protocole de purification.

### I.1. Isolement à partir des hémocytes des ascidies.

La récolte d'hémolymphe est effectuée, après lavage dans l'éthanol des ascidies (élimination des mucilages), par section au niveau du pied. La séparation des deux compartiments sanguins (le plasma et les hémocytes) se fait par centrifugation de l'hémolymphe (1000g pendant 10 minutes). On obtient ainsi les hémocytes au culot.

Le culot cellulaire est homogénéisé dans 10 volumes d'acide acétique 2 M en utilisant un homogénéisateur, puis l'homogénat est laissé pendant 12 heures sous agitation à 4°C. Ensuite, cet homogénat est centrifugé à 10000g pendant 20 minutes à 4°C. Le surnageant est alors soumis à un premier fractionnement sur cartouche Sep-Pak C18 de phase inverse (Sep-Pak Vac 12cc, Waters Corporation, USA, réf: WAT036915). Le surnageant est déposé sur la cartouche et trois fractions de polarité décroissante sont éluées grâce à 3 solutions préparées à partir d'eau ultrapure (EUP) et d'acétonitrile (ACN, HPLC Gradient Grade, ACROS Organics, réf: 32573-0025) additionnés de 0,05% d'acide trifluoroacétique (TFA, Fluka Chemika, réf: 91707):
10% d'ACN + 90% d'EUP + 0,05% TFA
60% d'ACN + 40% d'EUP + 0,05% TFA
80% d'ACN + 20% d'EUP + 0,05% TFA

Les extraits hémocytaires sont donc séparés en trois fractions. Ces différentes fractions sont ensuite congelées à - 80°C, lyophilisées et reprises dans 1 ml d'EUP additionnée de 0,05% de TFA. Une centrifugation est ensuite réalisée (10000g, 20 minutes à 4°C). Le surnageant constitue le matériel qui sera utilisé pour l'HPLC.

### I.2. Purification par HPLC.

Toutes les étapes de purification sont effectuées sur une HPLC de type Waters (modèle 1525 HPLC Binary Pump) équipée d'un détecteur spectrophotométrique (modèle 2487 Dual λ absorbance detector, Waters), d'un four thermostaté à effet Peltier (modèle 560-CIL, Cluzeau info labo) et relié à un système d'acquisition des données (logiciel Breeze). Les molécules éluées de la colonne sont analysées au niveau du détecteur UV à deux longueurs d'onde (224 et 280 nm).

La première étape d'HPLC s'applique à la fraction Sep-Pak 60% qui contient l'halocyntin. L'élution est effectuée en phase inverse sur une colonne Sephasil C18 (250*4.6mm, Waters, réf : WAT054275) suivant un gradient binaire linéaire: ACN et EUP additionnés de 0.05% TFA. Ce gradient varie de 2% à 72% d'ACN sur un temps de 90 minutes, le débit appliqué étant de 1ml /minute.

Les fractions éluées sont détectées par la visualisation de pics d'absorbance sur le moniteur et sont collectées dans des tubes de polyéthylène (tubes low-binding Minisorp, Merck eurolab, réf: 13183.01). Ces fractions seront ensuite congelées, lyophilisées et reprises dans de l'EUP TFA 0,05%.

Elles sont ensuite testées pour leurs activités antibactériennes. La fraction contenant l'halocyntin est soumise à une dernière étape de purification effectuée en phase inverse sur une colonne Sephasil C8 (150*2.1mm, Waters, réf : WAT056955). Le gradient d'élution utilisé encadre le pourcentage d'élution de la fraction concernée lors de l'étape de séparation précédente (31% ACN) : la fenêtre d'élution est étalée sur des pourcentages en acétonitrile de - 5% jusqu'à + 5% par rapport au pourcentage d'élution obtenu précédemment.

De plus, un étalement du gradient dans le temps (10 % en 40 minutes) est effectué afin d'obtenir une séparation plus fine. Cette seconde étape d'HPLC permet l'obtention du produit pur. L'élution est effectuée en 40 minutes, à un débit de 0.3ml/minutes, avec un gradient binaire linéaire: ACN/TFA et EUP/TFA.

### II. Caractérisation de l'halocyntin.

### II. 1. Tests d'activité antibactérienne au cours des étapes de purification.

Les tests d'activités antibactériennes, qui permettent de suivre l'activité biologique au cours des étapes de purification, sont réalisés en micro-plaque (96 puits, Becton Dickinson, USA, réf: 18572). Les différentes fractions récoltées après la séparation (Sep-Pak ou HPLC) sont congelées, lyophilisées puis reprises dans l'EUP/TFA.

Elles sont ensuite déposées à raison de 10 µl dans chaque puits de la micro-plaque et additionnées de 100 µl de culture bactérienne (*E. coli*) en milieu Poor Broth (1% bactotryptone, 0.5%NaCl, pH 7.5) dont la densité optique (DO) est amenée à 0.001. Le tout est placé sous agitation (250 rpm) à 37°C durant 12 heures.

### II.2. Caractérisation biochimique de l'halocyntin.

Après purification, une combinaison de techniques de spectrométrie de masse et de dégradation d'Edman, a permis à la Demanderesse d'obtenir la caractérisation biochimique complète de ce peptide. Il s'agit d'un peptide de 26 acides aminés dont la séquence suit : FWGHIWNAVKRVGANALHGAVTGALS (SEQ ID No. 1 dans la liste de séquences en annexe). Ce peptide est une molécule cationique (point isoélectrique estimé : 11) qui se structure vraisemblablement en hélice a amphipatique comme le suggèrent les prédictions de structure secondaire.

### II.3. Activité biologique de l'halocyntin.

Pour déterminer le spectre d'activité de l'halocyntin, des tests complémentaires ont été réalisés.

### i. Activité antibactérienne de l'halocyntin.

La concentration minimale bactéricide (MBC) pour chaque souche bactérienne testée est déterminée de la façon suivante : le peptide est repris dans une solution contenant 0.01% d'acide acétique et 0.2% de serumalbumine bovine (BSA) ; puis des dilutions sérielles de 2 en 2 sont réalisées dans la même solution 0.01% d'acide acétique et 0.2% de BSA. 10 µl de chaque dilution sont incubés dans des plaques 96 puits stériles (Becton Dickinson, USA, réf: 18572) en présence de 100 µl d'une suspension de bactéries ramenée à une densité optique de 0.001 à 600 nm dans le milieu Mueller Hinton (MHB, SIGMA, réf: M-9677).

La croissance bactérienne est contrôlée après 18 heures d'incubation sous agitation. La MBC est déterminée en étalant le contenu des trois premiers puits où aucune croissance bactérienne n'est observée ; cet étalement se fait sur des boîtes de Pétri coulées avec un agar préparé à partir du milieu de Mueller Hinton. Ces boîtes sont ensuite incubées 18 heures. La MBC correspond à la concentration la plus basse en peptide pour laquelle aucune colonie bactérienne n'est observée sur les boîtes incubées.

Le tableau 1 donne le spectre d'activité de la molécule. En résumé, l'halocyntin a une activité bactériolytique avec une action particulièrement puissante sur les bactéries à Gram positif.

**Tableau 1:**

| **Spectre d'activité de l'halocyntin** | |
|---|---|
| (Valeurs des MBC exprimées en µM) | |
| Bacteria | MBC |
| Bactéries à Gram-positif | |
| *M. luteus* | 1,56-3,13 |
| *S. aureus* | 6,25-12,5 |
| *B. megaterium* | 0,75-1,56 |
| *A*. *viridans* | 3,13-6,25 |
| *E. faecalis* | 6,25-12,5 |

| Bactéries à Gram-négatif | |
|---|---|
| *E. coli* DH5α | 6,25-12,5 |
| *S. typhimutium* | 50-100 |
| *F*. *aeruginosa* | >100 |
| *E. aerogenes* | 25-50 |
| *K. pneumoniae* | 12,5-25 |
| *N. gonorrhoeae* | 25-50 |

### i. Activité hémolytique de l'halocyntin.

Une quantité connue de peptide est reprise dans une solution contenant 0,01% d'acide acétique et 0,2% de serumalbumine bovine (BSA) de manière à obtenir une solution stock d'une concentration de 500 µM; puis des dilutions sérielles de cette solution sont réalisées dans la même solution 0,01% d'acide acétique et 0,2% de BSA (AA-BSA) . 20 µl de chacune de ces solutions ou 20 µl d'une solution Triton X-100 10% dans du PBS (contrôle 100% d'hémolyse) ou 20 µl de la solution AA-BSA (contrôle - peptide) sont ensuite ajoutés à 180 µl d'une solution contenant 2,5% (volume/volume) d'une suspension d'érythrocytes de mouton dans du PBS. Le mélange est ensuite incubé 30 minutes à 37°C, puis soumis à une centrifugation à 10000g pendant 3 minutes. Enfin, le surnageant est récupéré et son absorbance est mesurée à 600 nm. Le pourcentage d'hémolyse est ensuite calculé avec l'équation suivante : % hémolyse = (*A₆₀₀* de l'échantillon - *A₆₀₀* de contrôle-peptide)/ (*A₆₀₀* contrôle 100% d'hémolyse - *A₆₀₀* de contrôle-peptide)x100.

Comme présenté à la figure 1, l'halocyntin ne présente pas d'activité hémolytique jusqu'à des concentrations de 12,5 µM. Au delà de cette concentration, une très faible activité hémolytique est détectée. En effet, le peptide présente une activité hémolytique de 5,8 % sur les hématies de mouton à une concentration de 50 µM.

## Revendications

1. Peptide isolé dont la séquence en acides aminés est la suivante : FWGHIWNAVKRVGANALHGAVTGALS (SEQ ID No. 1 dans la liste de séquences en annexe), ses dérivés présentant au moins 70% d'identité avec la séquence ci-dessus, ses dérivés présentant une glycosylation, une amidation, une acylation, une acétylation, une méthylation ou portant un groupement protecteur et ses fragments d'au moins 7 acides aminés, lesdits dérivés et fragments présentant une activité antimicrobienne.

2. Polypeptide isolé comprenant un peptide selon la revendication 1.

3. Polynucléotide isolé **caractérisé en ce qu'**il code un peptide selon la revendication 1 ou un polypeptide selon la revendication 2.

4. Vecteur de clonage et/ou d'expression **caractérisé en ce qu'**il contient un polynucléotide selon la revendication 3.

5. Vecteur selon la revendication 4, **caractérisé en ce qu'**il contient en outre au moins un élément choisi dans le groupe des promoteurs constitutifs, des promoteurs inductibles, des éléments terminateurs.

6. Vecteur selon l'une quelconque des revendications 4 ou 5, **caractérisé en ce qu'**il comporte des éléments permettant son maintien et sa réplication dans un organisme hôte.

7. Organisme hôte, à l'exception de l'être humain, transformé à l'aide d'un vecteur selon l'une des revendications 4 à 6.

8. Organisme hôte, à l'exception de l'être humain, dans lequel un polynucléotide selon la revendication 3 est introduit pour la production d'un peptide selon la revendication 1 ou d'un polypeptide selon la revendication 2.

9. Organisme hôte selon l'une quelconque des revendications 7 ou 8, **caractérisé en ce qu'**il est choisi parmi les microorganismes, les cellules animales, les cellules végétales et les plantes.

10. Cellule végétale résistante aux maladies microbiennes comprenant un polynucléotide selon la revendication 3 et exprimant un peptide selon la revendication 1 ou un polypeptide selon la revendication 2.

11. Plante **caractérisée en ce qu'**elle comprend au moins une cellule végétale selon la revendication 10.

12. Utilisation d'un peptide selon la revendication 1 ou d'un polypeptide selon la revendication 2 pour la préparation d'une composition antimicrobienne et, plus particulièrement, antibactérienne destinée à lutter contre les bactéries à Gram positif.

13. Composition antimicrobienne comprenant à titre d'agent actif un peptide selon la revendication 1 ou un polypeptide selon la revendication 2 avantageusement associé dans ladite composition à un véhicule acceptable.

## Claims

1. Peptide isolated with the following sequence of amino acids: FWGHIWNAVKRVGANALHGAVTGALS (SEQ ID N° 1 in the appended list of sequences), its derivatives exhibiting at least a 70% identity with the above sequence, its derivatives exhibiting a glycosylation, an amidation, an acylation, an acetylation, a methylation, or containing a protective grouping and its fragments of at least 7 amino acids, said derivatives and fragments exhibiting anti-microbial activity.

2. An isolated polypeptide comprising a peptide according to Claim 1.

3. An isolated polynucleotide, **characterised in that** it codes a peptide according to Claim 1 or a polypeptide according to Claim 2.

4. A cloning and/or expression vector, **characterised in that** it contains a polynucleotide according to Claim 3.

5. A vector according to Claim 4, **characterised in that** it additionally contains at least one element chosen from the group of essential promoters, inducible promoters and terminator elements.

6. A vector according to Claim 4 or 5, **characterised in that** it comprises elements permitting its continuation in being and its replication in the host organism.

7. A host organism excepting a human being, converted with the help of a vector according to one of the Claims 4 to 6.

8. A host organism excepting a human being, in which a polynucleotide according to Claim 3 is introduced to produce a peptide according to Claim 1 or a polypeptide according to Claim 2.

9. A host organism according to Claim 7 or 8, **characterised in that** it is selected from among microorganisms, animal cells, vegetable cells and plants.

10. A vegetable cell resistant to microbial diseases, comprising a polynucleotide according to Claim 3 and expressing a peptide according to Claim 1 or a polypeptide according to Claim 2.

11. A plant **characterised in that** it comprises at least one vegetable cell according to Claim 10.

12. The use of a peptide according to Claim 1 or a polypeptide according to Claim 2 for the preparation of an anti-microbial composition and more particularly, anti-bacterial, destined to combat positive Gram bacteria.

13. An anti-microbial composition comprising as active agent a peptide according to Claim 1, or a polypeptide according to Claim 2, advantageously associated in said composition with an acceptable vehicle.

## Patentansprüche

1. Isoliertes Peptid mit der Aminosäuresequenz FWGHIWNAVKRVGANALHGAVTGALS (SEQ ID NR. 1 in der Sequenzliste in der Anlage), wobei seine Derivate zu mindestens 70 % mit der obigen Sequenz identisch sind, wobei seine Derivate eine Glycosylierung, eine Amidisierung, eine Acylierung, eine Acetylierung, eine Methylisierung aufweisen oder eine Schutzgruppe tragen und seine Fragmente mindestens 7 Aminosäuren, wobei die besagten Derivate und Fragmente eine antimikrobielle Aktivität aufweisen.

2. Isoliertes Polypeptid, ein Peptid nach Anspruch 1 umfassend.

3. Isoliertes Polynukleotid, **dadurch gekennzeichnet, dass** es ein Peptid nach Anspruch 1 oder ein Polypeptid nach Anspruch 2 kodiert.

4. Klonierungs- und/oder Expressionsvektor, **dadurch gekennzeichnet, dass** er ein Polynukleotid nach Anspruch 3 enthält.

5. Vektor nach Anspruch 4, **dadurch gekennzeichnet, dass** er außerdem mindestens ein Element enthält, das aus der Gruppe der konstitutiven Promotoren, der induktiven Promotoren, der Terminatoren ausgewählt ist.

6. Vektor nach einem der Ansprüche 4 oder 5, **dadurch gekennzeichnet, dass** er Elemente umfasst, die seinen Halt und seine Replikation in einem Wirtsorganismus erlauben.

7. Mit Hilfe eines Vektors nach einem der Ansprüche 4 bis 6 transformierter Wirtsorganismus, mit Ausnahme des Menschen.

8. Wirtsorganismus, mit Ausnahme des Menschen, in den ein Polynukleotid nach Anspruch 3 für die Produktion eines Peptids nach Anspruch 1 oder eines Polypeptids nach Anspruch 2 eingeführt ist.

9. Wirtsorganismus nach einem der Ansprüche 7 oder 8, **dadurch gekennzeichnet, dass** er aus den Mikroorganismen, den tierischen Zellen, den Pflanzenzellen und den Pflanzen ausgewählt ist.

10. Mikrobiellen Krankheiten gegenüber resistente Pflanzenzelle, die ein Polynukleotid nach Anspruch 3 umfasst und ein Peptid nach Anspruch 1 exprimiert oder ein Polypeptid nach Anspruch 2.

11. Pflanze, **dadurch gekennzeichnet, dass** sie mindestens eine Pflanzenzelle nach Anspruch 10 umfasst.

12. Verwendung eines Peptids nach Anspruch 1 oder eines Polypeptids nach Anspruch 2 für die Zubereitung einer antimikrobiellen und insbesondere antibakteriellen Zusammensetzung, die dazu bestimmt ist, gram-positive Bakterien zu bekämpfen.

13. Antimikrobielle Zusammensetzung, die als aktiven Wirkstoff ein Peptid nach Anspruch 1 oder ein Polypeptid nach Anspruch 2 umfasst, das in der besagten Zusammensetzung in vorteilhafter Weise mit einem akzeptablen Überträger verbunden ist.


### SEQUENCE LISTING

<110> Centre National de la Recherche Scientifique
   Université de Perpignan
<120> Peptide antimicrobien appelé halocyntin, gène codant ledit peptide, vecteur, organisme transformé et composition le contenant
<130> 30847/PCT
<140> PCT/FR04/XXXXX
   <141> 2004-03-05
<150> FR03/02715
   <151> 2003-03-05
<160> 1
<170> PatentIn version 3.1
<210> 1
   <211> 26
   <212> PRT
   <213> Halocynthia papillosa
<220>
   <221> PEPTIDE
   <222> (1)..(26)
   <223> Séquence en acides aminés de l'halocyntin
<400> 1
   Phe Trp Gly His Ile Trp Asn Ala Val Lys Arg Val Gly Ala Asn Ala
   1               5                   10                  15
   Leu His Gly Ala Val Thr Gly Ala Leu Ser
               20                  25